# EUROPEAN PATENT APPLICATION

(11) **EP 4 331 588 A1**
(43) Date of publication of application: **06.03.2024**
(21) Application number: 21939458.2
(22) Date of filing: 05.01.2022
(51) Int. Cl.: A61K 31/5415, A61K 31/542, A61P 21/00, A23L 33/10

(54) **COMPOSITION FOR PREVENTING OR TREATING MUSCULAR DISEASE, COMPRISING OXICAM-BASED COMPOUND**

(30) Priority: 27.04.2021 KR 20210054247; 25.10.2021 KR 20210142296
(71) Applicant: Animuscure Inc., Suwon-si, Gyeonggi-do 16419 (KR)
(72) Inventor: LEE, Sang-Jin, Seoul 04747 (KR); SO, Hyun-Kyung, Suwon-si, Gyeonggi-do 16362 (KR); LEE, Hye Young, Suwon-si, Gyeonggi-do 16319 (KR)
(74) Representative: Dehns
(86) International application number: PCT/KR2021/014993
(87) International publication number: WO 2022/231080

(57) **Abstract**

The present invention relates to a composition for the purpose of preventing, alleviating, or treating a muscular disease, comprising an oxicam-based compound, wherein the composition strengthens muscles and increases muscle mass through the effects of promoting differentiation of myoblasts and increasing muscle fibers, and can, thereby, have a treatment effect for various muscular diseases and have muscle strengthening or motor performance increasing effects.

## Description

### [Technical Field]

The present invention relates to a composition for preventing, alleviating or treating a muscular disease, comprising an oxicam-based compound.

### [Background Art]

Muscles play an important part in body functions such as energy metabolism and motor performance and may be damaged or weakened by various factors such as sarcopenia due to aging, muscular atrophy due to a nutritional imbalance or lack of exercise, other diseases such as cancer, and aging.

Sarcopenia, which is a major disease that damages muscles, is a disease in which muscle strength decreases as skeletal muscle mass decreases due to aging. The biggest feature of sarcopenia is a decrease in muscle mass, and the type of muscle fiber may also change. While Type I muscle fiber and Type II muscle fiber decrease at a similar rate with aging, the thickness of Type I muscle fiber decreases more noticeably in patients with sarcopenia. It has been reported that such sarcopenia causes muscle weakness and functional impairment in the elderly (Roubenoff R., Can. J. Appl. Physiol. 26, 78-89, 2001).

In addition, muscle atrophy, which is caused by a nutritional deficiency or long-term muscle disuse, occurs when the normal balance of protein synthesis and degradation is disrupted and thus proteins in muscles are degraded.

Various treatment methods for fundamentally treating these muscular diseases are being developed, and in particular, treatment methods using a mechanism that strengthens muscles or enhances muscle regeneration by promoting the differentiation of muscle cells from stem cells have been proposed. Since these methods can fundamentally treat muscular diseases, research on various therapeutic agents that enable this is required.

The inventors of the present invention conducted research on compounds having an effect of alleviating muscular diseases through the effects described above from various compounds, and thus completed the present invention.

### [Disclosure]

### [Technical Problem]

The inventors of the present invention have found that an oxicam-based compound promotes the differentiation of myoblasts, improves muscle energy metabolism to strengthen muscle strength, and in particular, has a therapeutic effect on muscular diseases such as muscle dystrophy, and thus completed the present invention.

The present invention is directed to providing a pharmaceutical composition for preventing or treating a muscular disease, comprising an oxicam-based compound or a pharmaceutically acceptable salt thereof.

Another object of the present invention is to provide a health functional food composition for preventing or alleviating a muscular disease, comprising an oxicam-based compound or a sitologically acceptable salt thereof.

Still another object of the present invention is to provide a composition for strengthening muscle strength, comprising an oxicam-based compound.

Yet another object of the present invention is to provide a composition for enhancing motor performance, comprising an oxicam-based compound.

Yet another object of the present invention is to provide a composition for promoting the differentiation of muscular stem cells, comprising an oxicam-based compound.

Yet another object of the present invention is to provide a composition for muscle regeneration, comprising an oxicam-based compound.

Yet another object of the present invention is to provide a composition for increasing muscle mass, comprising an oxicam-based compound.

Yet another object of the present invention is to provide a feed additive composition, comprising an oxicam-based compound.

### [Technical Solution]

One aspect of the present invention provides a pharmaceutical composition for preventing or treating a muscular disease, comprising an oxicam-based compound or a pharmaceutically acceptable salt thereof.

Another aspect of the present invention provides a health functional food composition for preventing or alleviating a muscular disease, comprising an oxicam-based compound or a sitologically acceptable salt thereof.

Still another aspect of the present invention provides a composition for strengthening muscle strength, comprising an oxicam-based compound.

Yet another aspect of the present invention provides a composition for enhancing motor performance, comprising an oxicam-based compound.

Yet another aspect of the present invention provides a composition for promoting the differentiation of muscular stem cells, comprising an oxicam-based compound.

Yet another aspect of the present invention provides a composition for increasing muscle mass, comprising an oxicam-based compound.

Yet another aspect of the present invention provides a feed additive composition, comprising an oxicam-based compound.

### [Advantageous Effects]

The present invention is a composition for preventing, alleviating, or treating a muscular disease, comprising an oxicam-based compound, and since the composition strengthens muscle strength and has a therapeutic effect on various muscular diseases by increasing muscle mass through effects of promoting the differentiation of myoblasts and regenerating muscular fibers, it can be used as a pharmaceutical composition or health functional food composition for this.

### [Description of Drawings]

FIG. 1 shows the results of confirming an effect of oxicam-based compounds on myoblast differentiation and comparing relative expression levels of Myo and eMHC when myoblasts are treated with each compound.
FIG. 2 shows the results showing the degree of differentiation of myoblasts (C2C12) according to the meloxicam administration concentration.
FIG. 3 shows the results of confirming an expression level of MHC, which is a myoblast differentiation marker, according to the meloxicam administration concentration.
FIGS. 4A and 4B show the results of confirming an expression level of MHC, which is a myoblast differentiation marker, according to meloxicam administration in human muscle stem cells at an age of 17 years, and FIGS. 5A and 5B show the results of confirming an expression level of MHC according to meloxicam administration in human muscle stem cells at an age of 66 years.
FIG. 6A shows the results of confirming the change in body weight upon meloxicam administration, and FIG. 6B shows the results of confirming an effect of increasing muscle mass through changes in weight of hindlimb muscle (TA, EDL, SOL, GAS) upon meloxicam administration.
FIG. 7A shows the results of confirming the change in body weight according to meloxicam administration, and FIG. 7B shows the results of confirming changes in weight of organs other than skeletal muscles.
FIGS. 8A and 8B show the results of confirming muscle regeneration and muscle fiber size through H&E staining when meloxicam was administered on days 4, 7, and 21, respectively, to a mouse model in which muscle damage had been induced by CTX.
FIG. 9A shows the results of confirming relative expression levels of muscle fiber types when meloxicam was administered to a mouse model in which a muscle damage had been induced by CTX; FIG 9B shows the results of confirming the size of a cross-sectional area of muscle fibers; and FIG. 9C shows the results of confirming the area of an expression type (MHCII type).
FIG. 10 shows a cross-sectional area (FIG. 10A) and a relative percentage (FIG. 10B) of muscle fibers in which GPDH, which is a metabolic enzyme (glycolytic enzyme) of muscles, is activated when meloxicam was administered to a mouse model in which muscle damage had been induced by CTX.
FIG. 11 shows the results of a grip strength test and a motor performance test when meloxicam was administered to a mouse model in which muscle damage had been induced by CTX.
FIG. 12 shows the results of comparing changes in body weight and blood glucose (FIG. 12A) and hindlimb muscle weight (FIG. 12B) when meloxicam was administered to 28-month-old mice.
FIG. 13 shows the results of comparing relative expression levels of muscle fiber types when meloxicam was administered to 28-month-old mice.
FIG. 14 shows the results of comparing changes in motor performance through a rotarod test when meloxicam was administered to 28-month-old mice.
FIG. 15 shows the results of comparing changes in body weight and grip strength (FIG. 15A) and motor performance (FIG. 15B) when meloxicam was administered to 14-month-old middle-aged mice.
FIG. 16 shows the results of comparing changes in body weight (FIG. 16A) and intake (FIG. 16B) when meloxicam was administered to 4-month-old young mice; and FIG 17 shows changes in grip strength and motor performance (FIG. 17A) and muscle mass compared to body weight (FIG. 17B).

### [Best Mode]

The present invention relates to a pharmaceutical composition for preventing or treating a muscular disease, comprising an oxicam-based compound or a pharmaceutically acceptable salt thereof.

The present invention relates to a health functional food composition for preventing or alleviating a muscular disease, comprising an oxicam-based compound or a sitologically acceptable salt thereof.

The present invention relates to a composition for strengthening muscle strength, comprising an oxicam-based compound.

The present invention relates to a composition for enhancing motor performance, comprising an oxicam-based compound.

The present invention relates to a composition for promoting the differentiation of muscular stem cells, comprising an oxicam-based compound.

The present invention relates to a composition for muscle regeneration, comprising an oxicam-based compound.

The present invention relates to a composition for increasing muscle mass, comprising an oxicam-based compound.

The present invention relates to a feed additive composition, comprising an oxicam-based compound.

Hereinafter, the present invention will be described in detail.

As one aspect of the present invention, the present invention may comprise an oxicam-based compound and a pharmaceutically acceptable salt thereof. The oxicam compound (oxicam-based compound) of the present invention is a compound widely used as a non-steroidal anti-inflammatory agent, and more specifically, may include lornoxicam, piroxicam, tenoxicam, anpyroxicam, droxicam, meloxicam, chlornotexicam, tenoxicam, and piroxicam, more preferably, meloxicam (Formula 1), piroxicam (Formula 2), tenoxicam (Formula 3) or lornoxicam (Formula 4), but is not limited thereto. The oxicam-based compound may be represented by the formulae below:

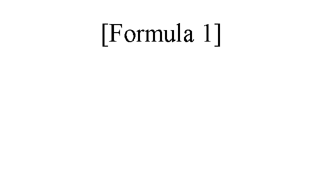

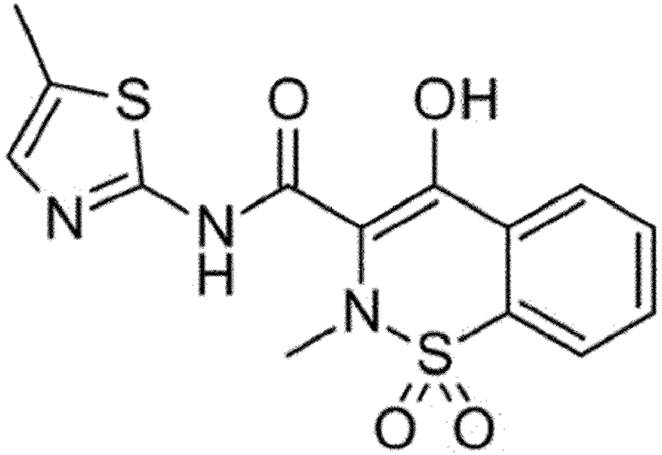

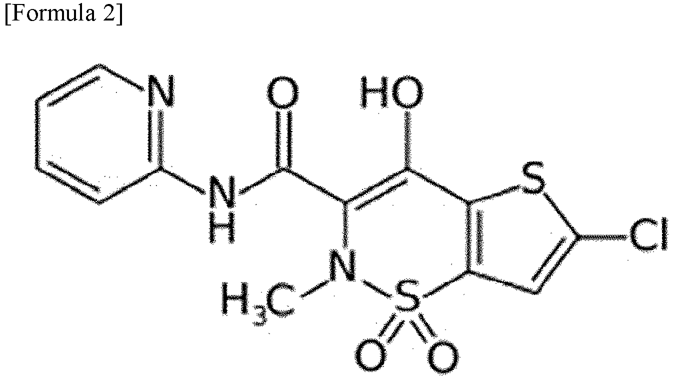

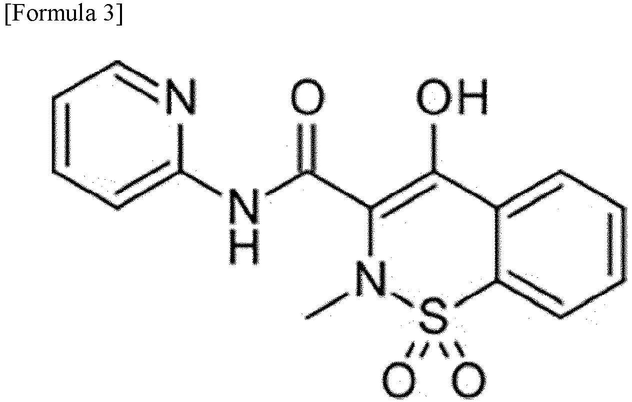

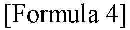

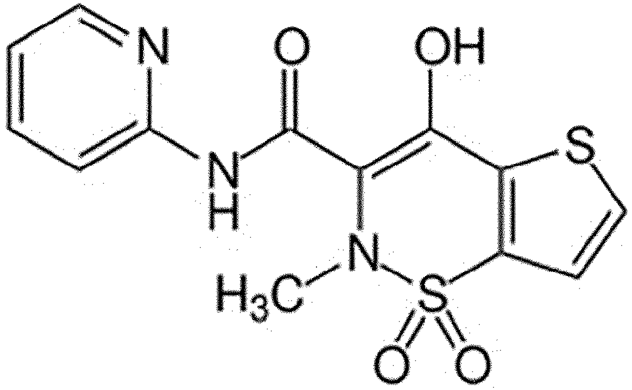

As one aspect of the present invention, the oxicam-based compound of the present invention has an effect of preventing or treating muscular diseases caused by aging, muscle dysfunction, muscle wasting, muscle degeneration or muscle damage. The "muscular disease" refers to a state in which muscle strength is weakened due to muscle damage or loss by aging or disease, which may be caused by various causes including genetic predisposition, age-related diseases such as high blood pressure, impaired glucose tolerance, diabetes, obesity, dyslipidemia, atherosclerosis and cardiovascular disease; chronic diseases such as cancer, autoimmune disease, infectious disease, AIDS, chronic inflammatory diseases, arthritis, malnutrition, renal disease, chronic obstructive pulmonary disease, pulmonary emphysema, rickets, chronic lower back pain, peripheral nerve injury, central nerve injury, and chemical damage; loss of motion due to causes such as fracture and trauma, or prolonged bed rest; and aging.

Although not limited thereto, the muscular disease comprises one or more muscular diseases selected from the group consisting of atony, muscular atrophy, muscle degeneration, myotonia, amyotrophic sclerosis, myasthenia gravis, cachexia, and senile sarcopenia, specifically, a muscle-related disease caused by senile muscular atrophy or cancer, more specifically, senile muscular atrophy or muscular atrophy caused by cancer, muscle degeneration, myotonia, amyotrophic sclerosis, myasthenia gravis, cachexia, senile sarcopenia, and muscle loss. Therefore, in one aspect, the muscular disease refers to a disease selected from the group consisting of atony, muscular atrophy, muscle degeneration, myotonia, amyotrophic sclerosis, myasthenia gravis, cachexia, and sarcopenia.

In the present invention, a preventive, therapeutic or alleviative effect on the muscular disease may be due to promotion of myoblast differentiation. In one embodiment of the present invention, it was confirmed that an oxicam-based compound promotes the differentiation of myoblasts. The "myoblast differentiation" refers to a process in which mononucleated myoblasts form multinucleated myotubes through fusion, and cells in the differentiation stage forming myotubes may be distinguished using markers such as Pax7-, MyoD+, and MyoG. The expression of myogenic transcription factors such as MyoD increases in cells in the early stage of differentiation forming myotubes, and MyoG increases in the middle stage. In the late stage of differentiation, the expression of a myosin heavy chain (MHC) increases. In an embodiment of the present invention, it was confirmed that expression of MHC and myogenin was increased when myoblasts were treated with meloxicam, which is an oxicam-based compound.

The oxicam-based compound of the present invention may improve motor performance. "Motor performance" refers to an ability to perform exercise using muscle strength, and at this time, muscle strength may be improved by increasing muscle mass, muscle endurance, and oxidative muscle mass and by improving muscle recovery and intramuscular energy balance, and may also be enhanced by decreasing intramuscular fatigue substances or the like. The oxicam-based compound of the present invention has an effect of enhancing motor performance through the various effects listed above on muscles. Regarding the effects described above, in one embodiment of the present invention, it was confirmed that actual muscle strength and motor performance were increased in a group administered meloxicam.

That is, the oxicam-based compound of the present invention has an effect of improving muscle function, such as increasing muscle mass, improving muscle strength, and increasing muscle recovery, and may have a preventive, therapeutic, and alleviative effect on muscle-related diseases.

The oxicam-based compound of the present invention may have a preventive, therapeutic, or alleviative effect on muscle diseases through muscle regeneration in an injured mouse. In an embodiment of the present invention, an effect of administering meloxicam to a muscle-damaged mouse model (CTX-injury) was confirmed. The results confirmed that muscle mass was increased compared to a control group, and when the degree of muscle and muscle fiber regeneration was observed when meloxicam was administered, it was confirmed that muscle and muscle fiber were regenerated more densely. In addition, when an oxicam-based compound such as meloxicam was administered, it was confirmed that the ratio of Type II muscle fibers (MHC Type II) was greatly increased compared to a control group. In addition, in not only muscles damaged by CTX but also muscle tissues of mice damaged or weakened by aging, there may be an effect of improving muscle strength and motor performance through effects of promoting the differentiation of muscle stem cells, increasing muscle fiber size, and increasing muscle mass.

In addition, the oxicam-based compound of the present invention may have an effect of increasing muscle strength and improving muscle function by not only regenerating muscles damaged by muscle diseases or aging but also an effect of regenerating muscles and increasing muscle mass in normal muscles. In an embodiment of the present invention, an effect of differentiating muscle stem cells and increasing muscle mass was confirmed in young mice (14-month-old and 4-month-old), and it was confirmed that when meloxicam is administered for a long time even in young muscles, there is an effect of increasing muscle strength and improving motor performance.

The composition of the present invention may be used for various uses such as pharmaceuticals, health functional food, functional food, animal feed, and cell culture compositions and may promote the differentiation of myoblasts or increase mitochondrial activity in muscles, thus having a preventive, alleviative or therapeutic effect on a muscular disease.

The term "prevention" used in the specification refers to all actions that can suppress or delay the onset of a muscle disease by administration of the pharmaceutical composition according to the present invention.

The term "treatment" used in the specification refers to all actions that ameliorate or beneficially change symptoms by administration of the pharmaceutical composition according to the present invention.

The pharmaceutical composition of the present invention is used after formulating into forms of oral formulations such as powder, granules, tablets, capsules, suspensions, emulsions, syrup, and aerosol, external preparations, suppositories, and sterile injection solutions according to conventional methods, and may further include a carrier or excipient necessary for the formulation. Pharmaceutically acceptable carriers, excipients, and diluents that may be further included in addition to the active ingredient include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, magnesium stearate, and mineral oil. When formulated, it is prepared using diluents or excipients such as commonly used fillers, extenders, binders, wetting agents, disintegrants, and surfactants.

For example, solid preparations for oral administration include tablets, pills, powder, granules, capsules, and the like, and these solid preparations are prepared by mixing the extract or compound with at least one excipient such as cotton, starch, calcium carbonate, sucrose or lactose, gelatin, and the like. In addition to simple excipients, lubricants such as magnesium citrate and talc are also used. Liquid preparations for oral administration include a suspension, an internal solution, an emulsion, syrup, and the like, and in addition to water and liquid paraffin, which are commonly used simple diluents, various excipients, for example, wetting agents, sweeteners, flavoring agents, and preservatives may be included.

Formulations for parenteral administration include sterilized aqueous solutions, non-aqueous solvents, suspensions, emulsions, freeze-dried formulations, and suppositories. As non-aqueous solvents and suppositories, propylene glycol, polyethylene glycol, vegetable oils such as olive oil, and injectable esters such as ethyl oleate may be used. As a base for suppositories, Witepsol, Macrogol, Tween 61, cacao butter, laurin butter, glycerol, gelatin, and the like may be used.

The pharmaceutical composition of the present invention may be administered orally or parenterally (intravenously, subcutaneously, intraperitoneally or topically applied) according to a desired method, and a dosage depends on a patient's condition and body weight, the degree of a disease, the type of drug, and the administration route and time and may be selected in an appropriate form by those skilled in the art.

The pharmaceutical composition of the present invention is administered in a pharmaceutically effective amount. In the present invention, "pharmaceutically effective amount" means a reasonable amount applicable to medical treatment, which is an amount sufficient to treat a disease, wherein the standard may be determined according to a patient's disease, severity of disease, drug activity, sensitivity to a drug, administration time, administration route and excretion rate, treatment duration, concomitantly used ingredients, and other factors. The pharmaceutical composition of the present invention may be administered as an individual therapeutic agent or in combination with other therapeutic agents, and may be administered sequentially or simultaneously with conventional therapeutic agents. By considering all of the factors described above, the dosage may be determined at a level that can minimize side effects, which may be easily determined by those skilled in the art. Specifically, the dosage of the pharmaceutical composition may vary depending on a patient's age, body weight, severity of disease, sex, or the like, and generally an amount of 0.001 to 150 mg, preferably 0.01 to 50 mg, and more preferably 0.01 to 5 mg per 1 kg of body weight may be administered every day or every other day, 1 to 3 times per day. However, this is exemplary, and the dosage may be set differently according to need.

In addition, the composition of the present invention may be food or health functional food, and in particular, the "health functional food" refers to food that is manufactured and processed using raw materials or components having functionality that is useful to the human body according to Health Functional Foods Act No. 6727, wherein "functional" refers to intake for the purpose of obtaining useful effects for health purposes, such as adjusting nutrients for the structure and functions of the human body or physiological functions.

The food or health functional food of the present invention may be manufactured and processed into a pharmaceutical administration form such as powder, granules, tablets, capsules, pills, suspensions, emulsions, and syrup or a health functional food such as tea bags, leached tea, beverages, candy, jelly, and gum for the purpose of preventing and alleviating a muscular disease.

The food or health functional food composition of the present invention may be used as a food additive, and may be commercialized alone or in combination with other ingredients. In addition, nutrients, vitamins, electrolytes, flavoring agents, coloring agents and enhancers, pectic acid and a salt thereof, alginic acid and a salt thereof, organic acids, protective colloidal thickeners, pH adjusters, stabilizers, preservatives, glycerin, alcohols, and carbonation agents used for carbonated beverages may be included. The ingredients described above may be used alone or in combination, and may be used in combination in an appropriate amount.

As an embodiment of the present invention, the present invention relates to a composition for strengthening muscle strength, comprising an oxicam-based compound.

The "strengthening of muscle strength" refers to effects of enhancing physical performance, enhancing maximum endurance, increasing muscle mass, enhancing muscle recovery, reducing muscle fatigue, improving an energy balance, or a combination thereof. The composition may increase total muscle mass by increasing muscle mass through an ability to differentiate myoblasts into muscle cells, and may enhance maximum endurance, thereby enhancing physical performance and reducing muscle fatigue. In addition, since muscle cells may be quickly replaced, muscle damage may quickly heal. In addition, the composition for strengthening muscle strength of the present invention may be prepared in the form of a food composition or food additive, and in particular, may be prepared in the form of a health food composition. The food composition is as described above. Therefore, the composition for strengthening muscle strength of the present invention may be used in the form of a supplement for not only muscle loss due to aging but also for muscle generation and muscle strengthening in common people.

As another aspect of the present invention, the present invention relates to a feed or feed additive composition comprising an oxicam-based compound.

In the present invention, 'feed' refers to a material that supplies organic or inorganic nutrients necessary for maintaining the life of an animal. The feed may include nutrients such as energy, proteins, lipids, vitamins, and minerals required by animals such as livestock, and may include vegetable feed such as grains, roots and fruits, food processing by-products, algae, fibers, oils, starches, gourds, and grain by-products or animal feed such as proteins, inorganic materials, oils, fats and oils, and single-cell proteins, but is not limited thereto.

In the present invention, 'feed additive' refers to a substance that is added to feed to improve the productivity or health of animals, and in this case, the feed additive may further include amino acids, vitamins, enzymes, flavoring agents, silicates, buffering agents, extracts, oligosaccharides, and the like for the purpose of promoting growth and preventing diseases, but it is not limited thereto.

### [Modes of the Invention]

Hereinafter, embodiments will be described in detail to specifically describe the present specification. However, the embodiments according to the present specification may be modified into many different forms, and the scope of the present specification is not construed as being limited to the embodiments described below. The embodiments described in this specification are provided to more completely explain the present specification to one of ordinary skill in the art.

### <Example 1> Isolation and culture of muscle cells

C2Cl2, which is a myoblast line obtained from live mice of the C3H species, is widely used in studies on muscle cell differentiation. The C2C12 cells were cultured in a general cell culture medium and a differentiation medium, respectively. DMEM supplemented with 10% fetal bovine serum was used as a normal cell culture medium (i.e., a growth medium (GM)), and DMEM containing 2% horse serum was used as a differentiation medium (DM).

In addition, human muscle stem cells (skMDC human skeletal muscle cells (Standard Donors), Cat NO: SK-1111) isolated from the rectus abdominis of 17- and 66-year old Caucasian males were purchased from MyoSite and used.

### <Example 2> Preparation of muscle injury (CTX-injury) mouse model

As experimental animals, 30 five-month old C57BL/6 male mice were used. Five animals each of the experimental animals having a similar body weight were assigned to classify a control group to which meloxicam was not administered and an experimental group to which meloxicam was administered. Meloxicam was orally administered to the mice of the experimental group at a dose of 0.02 mg/kg for 7 days. Then, 50 µl of 20 µM cardiotoxin (CTX) was directly injected into the tibialis anterior (TA) muscle to induce muscle damage, and meloxicam (0.02 mg/kg) was orally administered for 21 days.

### <Experimental Example 1> Confirmation of myoblast differentiation enhancement effect

### 1-1. Mouse muscle stem cell differentiation enhancement effect

The C2Cl2 cell line of Example 1 was seeded into a cell culture medium and cultured in a DMEM medium for 24 hours, and then a differentiation medium was treated with meloxicam (F03), lornoxicam (F03-1), piroxicam (F03-2) and tenoxicam (F03-3), which are oxicam-based compounds, to analyze the relative expression levels of myogenin and a myosin heavy chain (MHC), which are differentiation markers. After the C2C12 cells induced to differentiate in Example 1 were collected, lysed by adding a lysis buffer (20 mM Tris-HCl, pH 8.0, 150 mM NaCl, 1% Triton X, proteinase inhibitor), cytolytic samples was quantified, and then the same amount of protein was subjected to SDS-PAGE electrophoresis and transferred to a PVDF membrane. The membrane was blocked with 5% skim milk and washed with TTBS (0.03% Tween 20, Tris 2.42 g, NaCl 9 g, pH 7.41 L). An MHC (myosin heavy chain) primary antibody, which is a differentiation marker, diluted 1:500 in TTBS containing 5% BSA was added and allowed to react at 4°C overnight. Afterward, a secondary antibody diluted 1:5000 in TTBS containing 5% skim milk was added and allowed to react at room temperature, and then an enhanced chemiluminescent solution (ECL, Pierce) was added. Then, the membrane was exposed to an X-ray film to confirm protein expression levels.

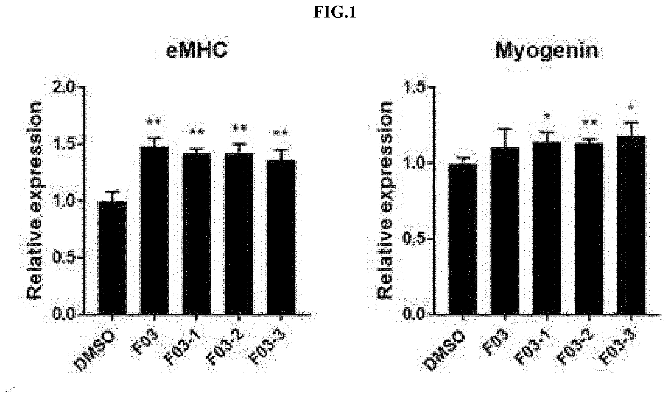

As a result, as shown in FIG. 1, it was confirmed that, when the oxicam-based compounds were treated, compared to the control group (DMSO), the relative expression level of the differentiation markers of myoblasts was increased.

Among the compounds described above, C2C12 cells were treated with meloxicam at different concentrations of 1, 10, 100, 1,000, and 10,000 nM, respectively, and differentiation was induced for 3 days while changing the medium every other day. After differentiation, the cells were observed under a microscope, and the cells were disrupted and subjected to Western blot analysis using myogenin and MHC antibodies, which are differentiation markers. The C2C12 cells were washed with 1XPBS, fixed with 4% paraformaldehyde at room temperature, and then allowed to react at room temperature after adding a permeabilization buffer. The reaction was performed with PBST (blocking buffer) containing 5% goat serum and PBS containing 0.05% Tween 20 to inhibit non-specific antibody binding. After adding a primary antibody against myosin heavy chain (MHC), the resulting mixture was allowed to react at room temperature. A secondary antibody diluted 1:5000 in a blocking buffer was added and allowed to react at room temperature, and after applying a mounting solution (40% glycerol in DW) and fixing, images were taken under a fluorescence microscope to analyze the results.

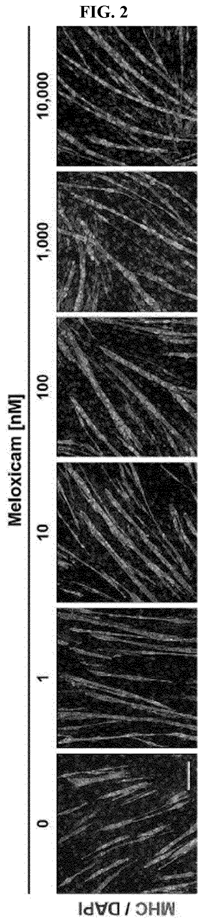

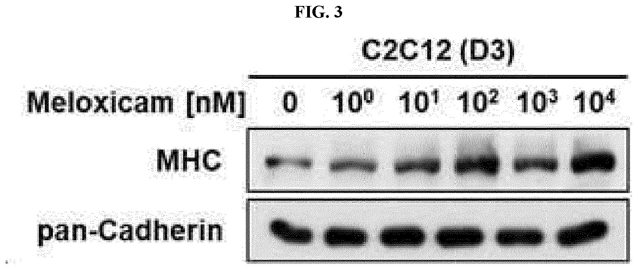

As a result, as shown in FIG. 2, MHC was clearly observed in the myoblasts treated with a high concentration of meloxicam, and as shown in FIG. 3, it was confirmed that the expression level was also increased.

### 1-2. Human muscle stem cell differentiation enhancement effect

An experiment was conducted to confirm whether meloxicam has an effect of promoting differentiation in human muscle stem cells.

Human muscle stem cells (skMDC human skeletal muscle cells (Standard Donors), Cat NO: SK-1111) isolated from the rectus abdominis of 17- and 66-year old Caucasian males were purchased from MyoSite and used. Human muscle stem cells were seeded in a cell culture medium and cultured for 24 hours. Then, the differentiation medium was treated with meloxicam (1 µM), which is an oxicam-based compound, and differentiation into muscle fibers was induced for 4 days. The degree of differentiation into muscle fibers was measured through MHC immunostaining, which is a muscle fiber marker. Differentiation was analyzed by muscle fiber diameter.

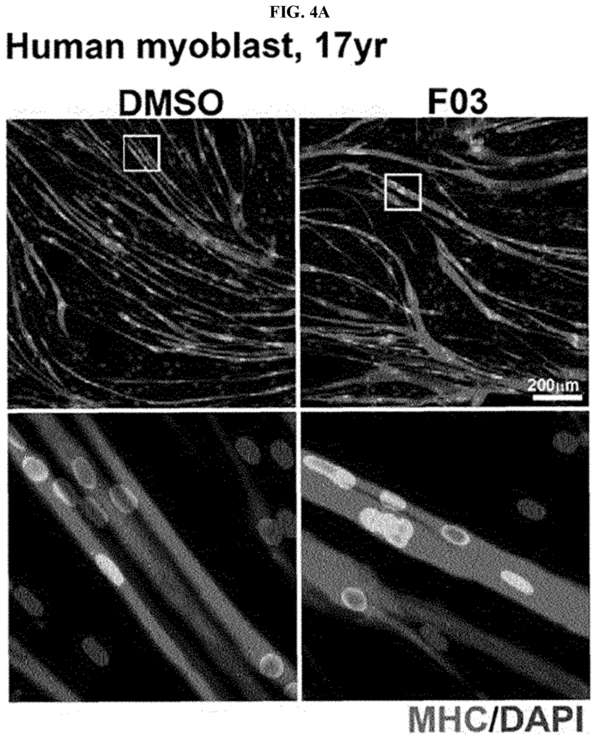

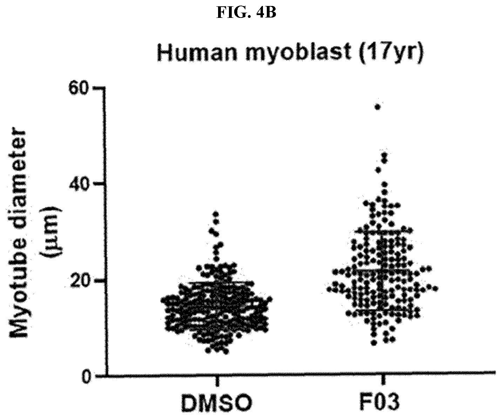

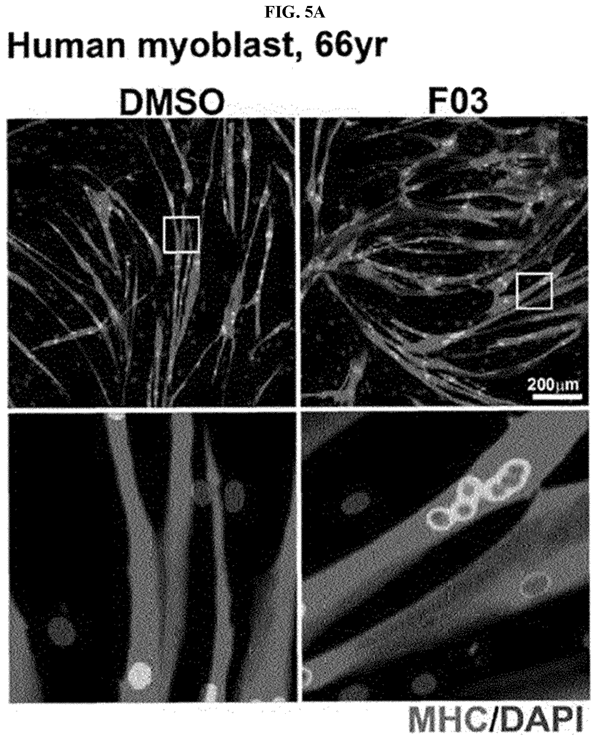

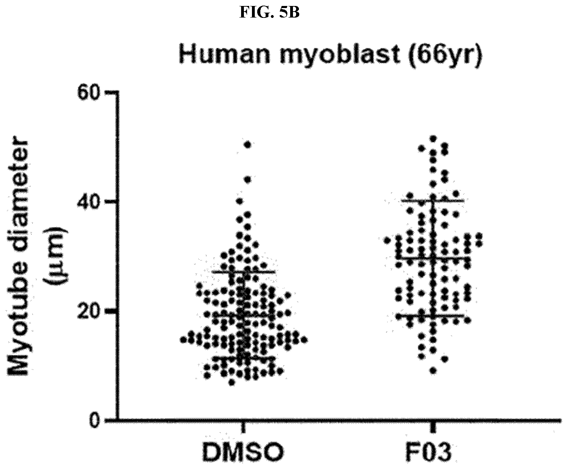

As a result, as shown in FIG. 4, in the case of the muscle stem cells extracted from a 17-year-old subject, it was confirmed that the myotube diameter increased, and as shown in FIG. 5, in the case of the muscle stem cells extracted from a 66-year-old subject, it was confirmed that both expression and the myotube diameter were increased.

### <Experimental Example 2> Confirmation of muscle recovery effect in muscle-damaged mouse model

### 2-1. Effect of increasing muscle mass in muscle-damaged mouse model

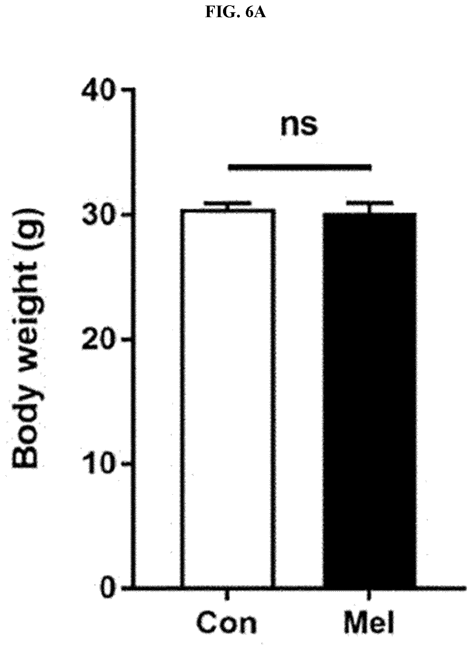

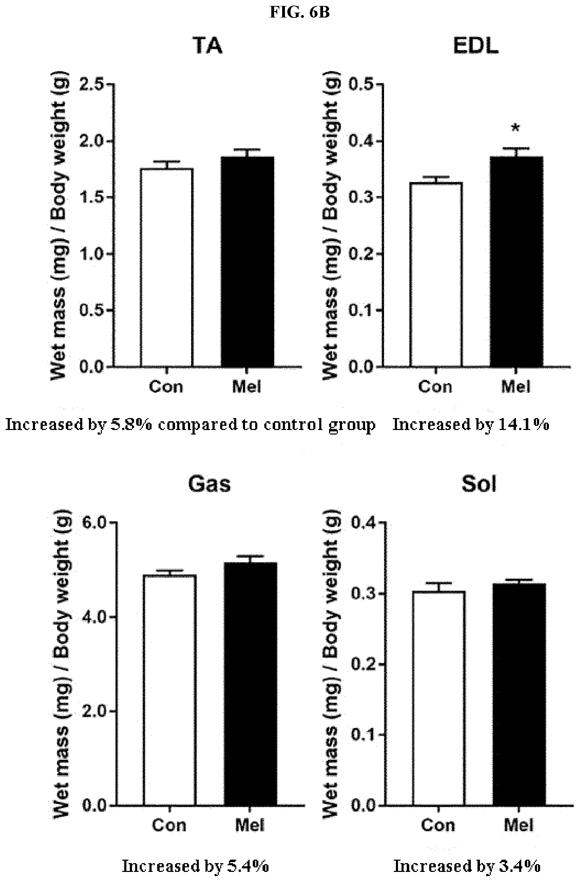

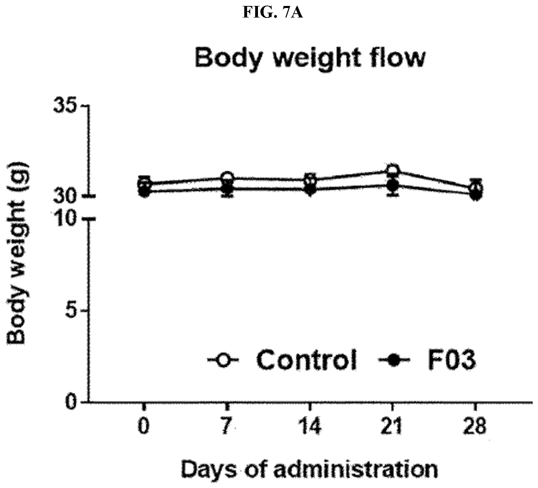

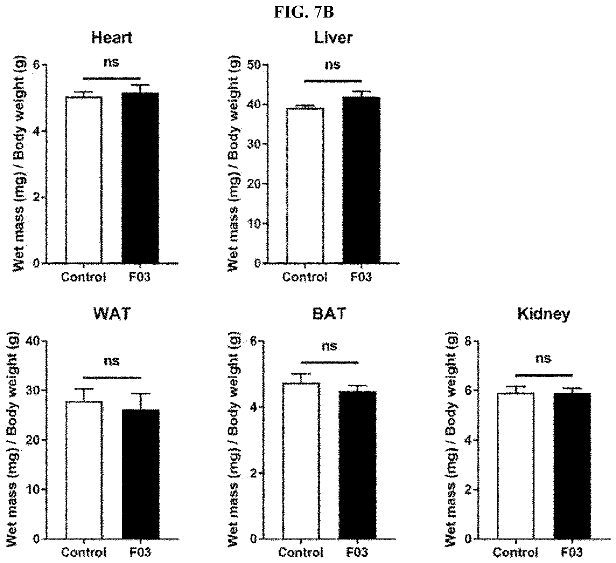

An experiment was performed to confirm the muscle recovery effect by using a CTX-injured mouse model. After administration of meloxicam for 21 days after CTX injury was induced, the weight of each of the tibialis anterior muscle (TA), extensor digitorum longus muscle (EDL), soleus muscle (Sol), and gastrocnemius muscle (Gas), which are muscle tissues of the hindlimb, was measured and compared, and as a result, it was confirmed that muscle mass was increased by 5.8% in TA, 14.1% in EDL, 5.4% in Gas, and 3.4% in Sol, respectively, compared to the control group (FIG. 6). These results contrast with the result that there was little change in the body weight and the weight of other organs during the same period (FIG. 7), indicating that meloxicam specifically increases the amount of skeletal muscle.

### 2-2. Effects of muscle regeneration and increasing muscle fiber size in muscle-damaged mouse model

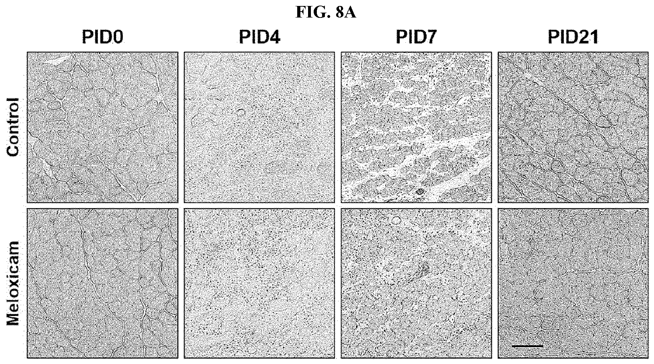

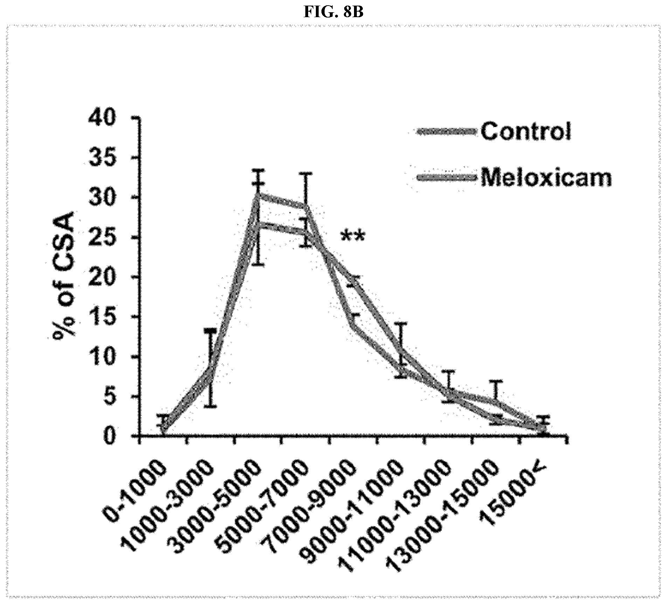

For the CTX-injured mouse model, to confirm the effect of meloxicam on muscle regeneration and increasing muscle fiber size, hematoxylin & eosin staining (H&E staining) was performed using the TA muscle isolated from the tissue of each of the experimental animals. Frozen sections fixed with 4% paraformaldehyde were stained with hematoxylin and stained with eosin for counterstaining and fixed with a mounting solution, and then observed under an optical microscope. As shown in FIG. 8, it was confirmed that muscle recovery and the size of the muscle fibers were increased from day 4, day 7 and day 21 of recovery after muscle damage.

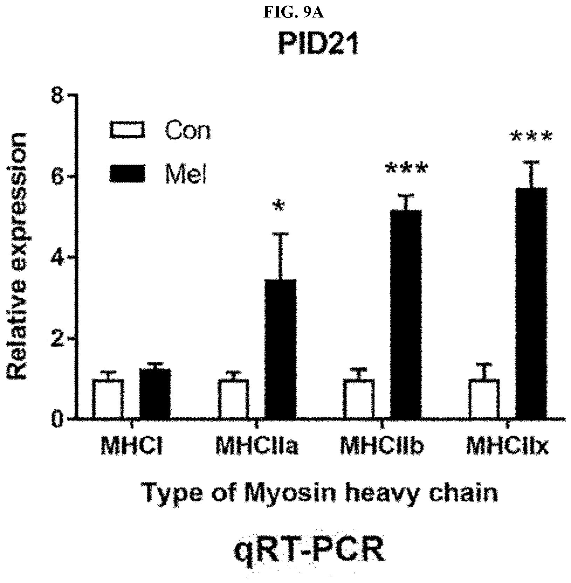

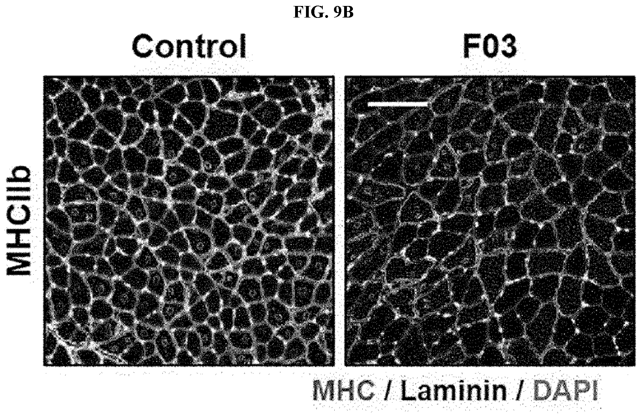

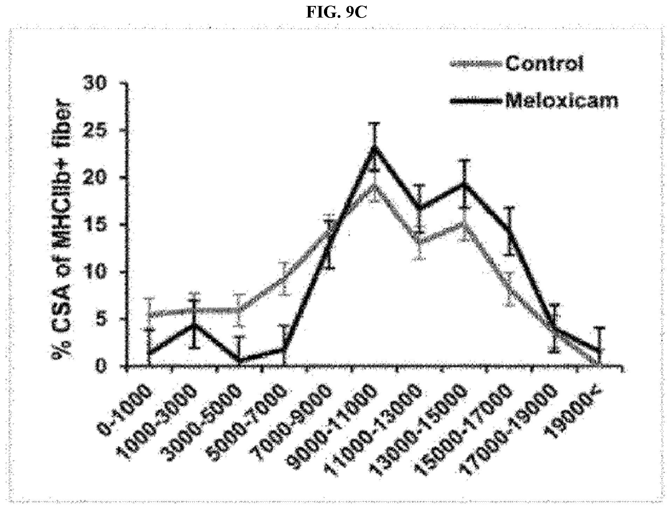

In addition, to additionally confirm the increased muscle type, the relative expression levels of mRNA related to muscle type were compared. As a result, as shown in FIG. 9, it was confirmed that the amount of Type II muscle fiber was significantly increased in the meloxicam-administered group, especially on day 21 after damage, compared to the control group.

Furthermore, for muscle fiber size analysis, immunohistochemical staining was performed on the TA muscle tissue using a laminin antibody and the tissue was observed under a fluorescence microscope, and as shown in the photo at the bottom of FIG. 9, it can be seen that the cross-sectional area (CSA) of the muscle fibers shown by laminin staining increased, and the CSA of metabolic muscle fibers expressing MHC was observed to be greater. Through this, it was confirmed that the increased muscle fibers and the resulting increase in muscle mass are due to increases in the diameter and percentage of metabolic muscle fibers.

### 2-3. Increased activity of metabolic enzymes in muscle of muscle-damaged mouse model

Meloxicam was administered to the CTX-injured mouse model on day 21 after injury to confirm the effect of increasing glycolytic enzyme activity in muscular mitochondria in the administration group.

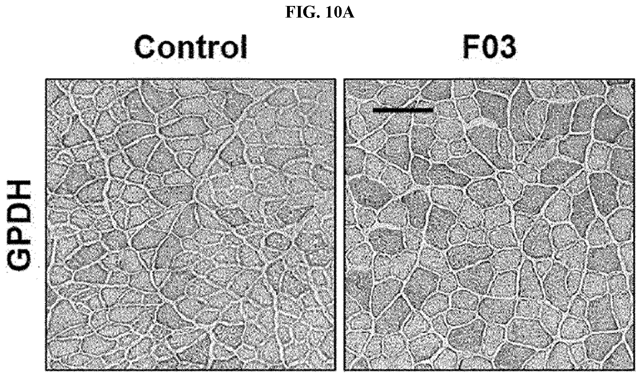

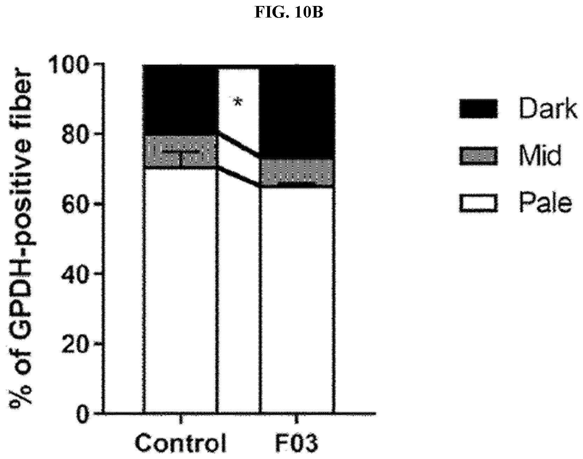

Alpha-glycerol phosphate, which is a substrate of the enzyme glycerol-3-phosphate dehydrogenase (GPDH), was added to the TA muscle sections isolated from the tissues of the experimental animals, and the color change of muscle fibers was confirmed according to the activity of GPDH. The changed muscle sections were fixed with Aquatex (Merck) and observed under a microscope. As a result, as shown in FIG. 10, in the case of the meloxicam-administered group, it was confirmed that the metabolic muscle fiber size increased (left), and it was confirmed that the percentage of muscle fibers in which GPDH was activated was higher than that of the control group. From the above results, it can be seen that the activity of metabolic enzymes in muscle is increased by administration of meloxicam.

### 2-4. Effect of increasing muscle strength and motor performance in muscle-damaged mouse model

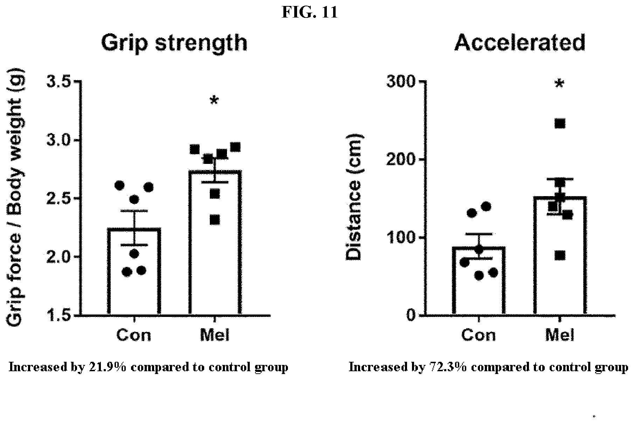

On day 21 after inducing CTX-injury, to confirm the muscle strength and motor performance of the meloxicam-administered group and the control group, an experiment for evaluating muscle strength and motor performance was performed. A grip strength test was performed to confirm the muscle strength of the administration group and the control group. The grip strength test was performed using a mouse grip strength tester manufactured by BIOSEB. A mouse was placed on a wire mesh attached to an instrument panel capable of monitoring the strength of the force, and the force with the mouse gripped the wire mesh was measured while pulling its tail. An average value of the measurements obtained by repeating the measurement four times in succession was divided by body weight. As a result, as shown in FIG. 11, it was confirmed that the muscle strength increased by about 21.9% in the meloxicam-administered group compared to the control group, and the experiment for evaluating motor performance also confirmed that motor performance increased by up to 72.3% in the meloxicam-administered group. A rotarod test was performed to confirm the motor performance of the administration group and the control group. In the rotarod test, measurement was performed using a rotarod device for mice manufactured by Ugo Basile. The distance until a mouse falls off from the rotating rotor is measured. The measurement in the rotarod test was performed after adaptation training, which was conducted at 8 rpm for 5 minutes, at 10 rpm for 3 minutes, and at 13 rpm for 1 minute for two days. In a constant rotarod test, the measurement was performed at 13 rpm until a mouse fell off, and in an accelerated rotarod test, the measurement was performed at an acceleration from 5 rpm to 18 rpm in 40 seconds. In both experiments, measurements were made based on a maximum value up to 500 seconds, and the experiments were stopped at 500 seconds.

### <Experimental Example 3> Confirmation of effect of increasing muscle mass and motor performance according to age upon long-term meloxicam administration

### 3-1. Changes in muscle mass, muscle type and motor performance in aging mice

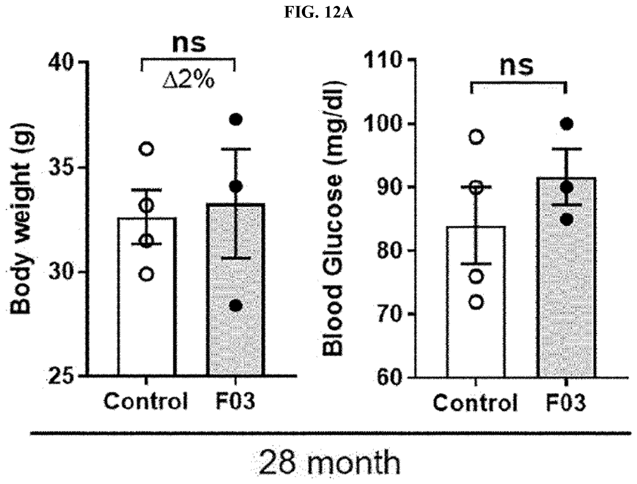

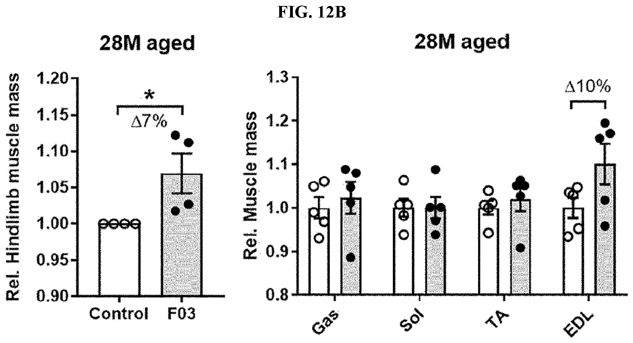

After administrating meloxicam to 24-month-old mice for 4 months, changes in hindlimb and skeletal muscle mass were analyzed by measuring weight and compared. As a result, as shown in FIG. 12, it was confirmed that muscle mass increased in the hindlimb muscles and EDL muscle (bottom), indicating that muscle mass increased at a relatively higher rate compared to the total weight gain (top).

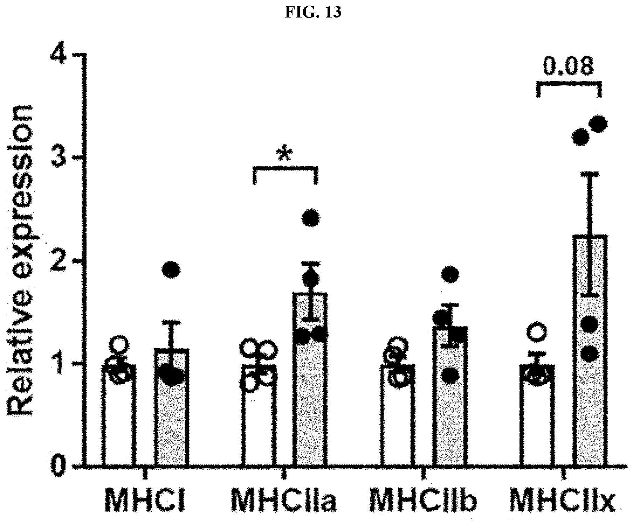

In addition, the relative expression level of mRNA for the expressed muscle type was confirmed, and as shown in FIG. 13, the expression rate of Myh Type II muscle fibers was especially increased in the meloxicam-administered group. That is, it was confirmed that muscle mass and the percentage of metabolic muscle fibers increased in the aged mice administered meloxicam.

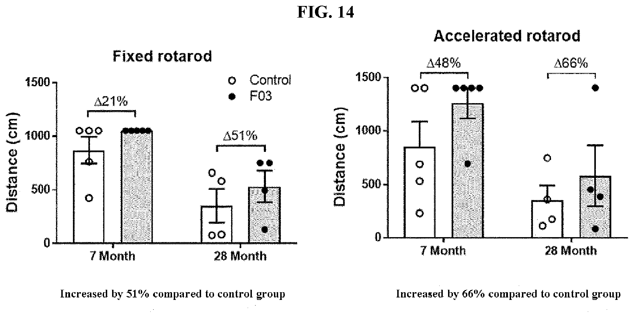

In addition, to confirm changes in motor performance of the meloxicam-administered group and the control group, a motor performance test was performed with a rotarod apparatus. As a result, as shown in FIG. 14, it was confirmed that the motor performance of the meloxicam-administered group increased by more than 50%, compared to the control group. Meanwhile, the effect of increasing motor performance was confirmed not only in the aged mice but also in 7-month-old mice. Therefore, it was confirmed that the motor performance improving effect by meloxicam was found not only in aged muscles but also in normal muscles.

### 3-2. Changes in muscle strength and motor performance in mice (4 months old)

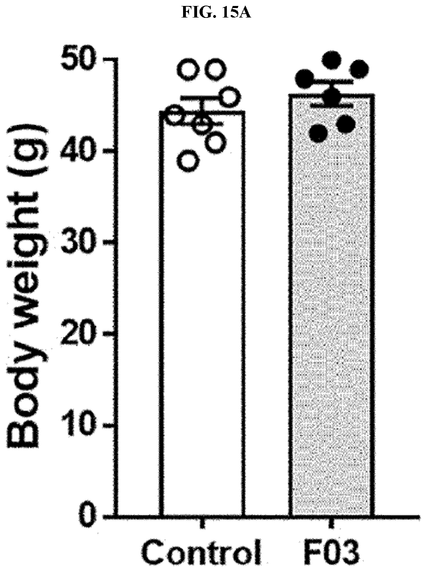

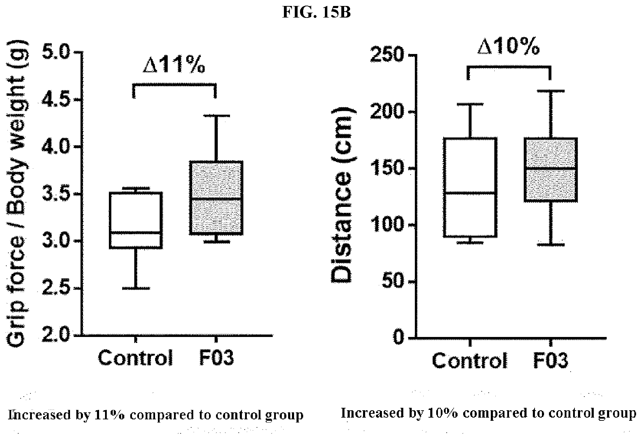

To measure changes in muscle strength and motor performance in relatively young mice by the administration of meloxicam, an experiment was performed. On day 21 after inducing CTX-injury, a grip strength test for measuring the muscle strength of the meloxicam-administered group and the control group and an experiment for confirming motor performance were conducted. The grip strength test was performed by using a mouse grip strength tester manufactured by BIOSEB. A mouse was placed on a wire mesh attached to an instrument panel capable of monitoring the strength of the force, and the force with the mouse gripped the wire mesh was measured while pulling its tail. An average value of the measurements obtained by repeating the measurement four times in succession was divided by body weight. As a result, as shown in FIG. 15, it was confirmed that muscle strength increased by about 21.9% in the meloxicam-administered group compared to the control group, and the experiment for evaluating the motor performance also confirmed that motor performance increased by up to 72.3% in the meloxicam-administered group.

### 3-3. Changes in muscle strength and motor performance in normal mice (4 months old) upon long-term administration

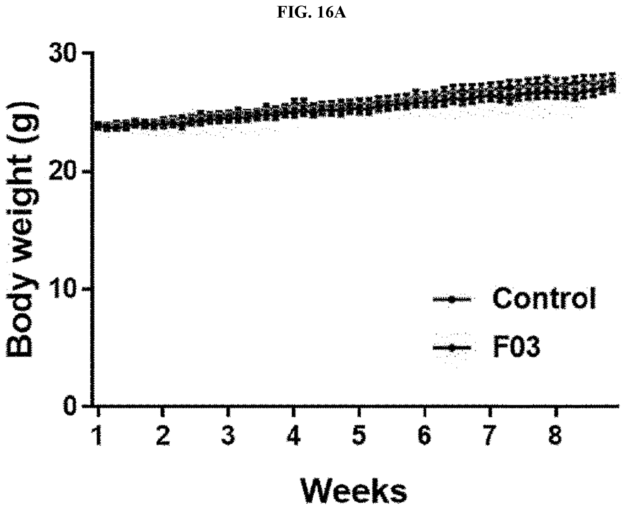

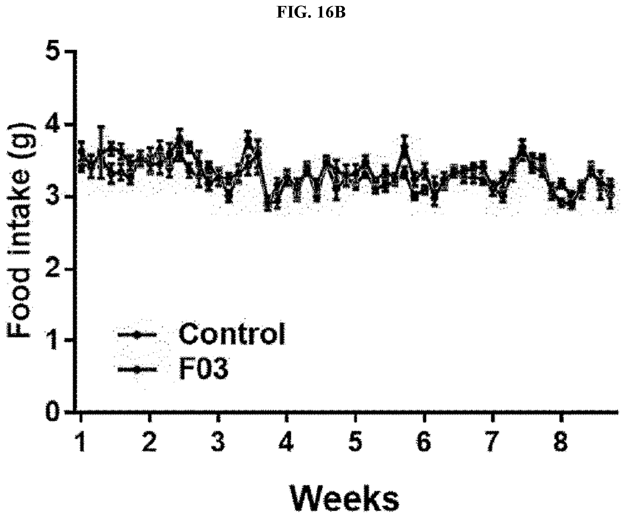

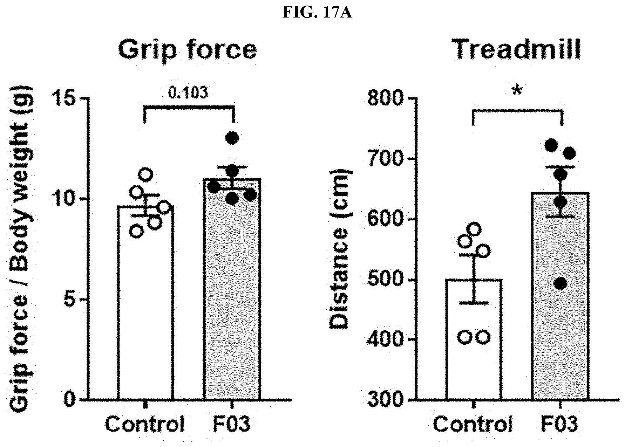

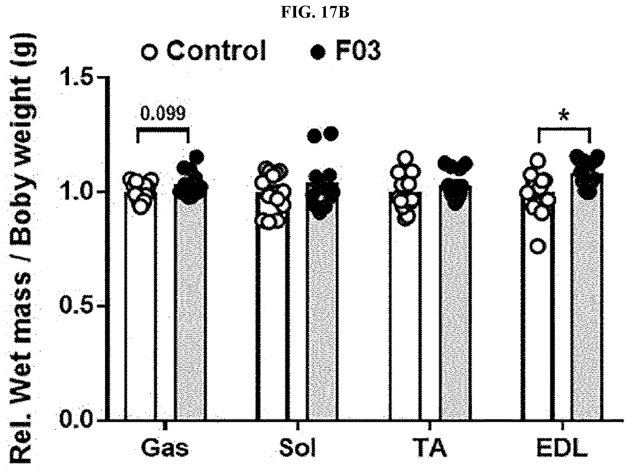

An experiment was performed with 4-month-old normal mice to observe changes in muscle strength and motor performance upon long-term meloxicam administration. First, after administering meloxicam to normal mice for 2 months, as a result of comparing the body weight change with that of the non-administration group, no significant change was observed (FIG. 16A), and no change in intake was observed (FIG. 16B). A grip strength test and a treadmill test were conducted to confirm the motor performance of the administration group and the control group. In the treadmill test, measurement was performed by using a treadmill device for mice manufactured by Columbus Instruments. Measurements started at a 10% slope and 8 m/min, which was increased 1 m/min every 2 minutes, and continued until the mouse became tired. As a result of the grip strength test, it was confirmed that muscle strength was improved in the meloxicam administration group, and the exercise duration was also improved compared to the non-administration group (FIG. 17A). In addition, it was confirmed that the muscle mass-to-body weight ratio increased more in the meloxicam administration group than in the control group (FIG. 17B). Therefore, from the experimental results described above, it was found that when meloxicam is administered, muscle function is improved and thus muscle strength and motor performance are increased even in normal young mice.

In the foregoing, the present invention has been described with respect to its preferred embodiments. Those of ordinary skill in the art to which the present invention pertains will understand that the present invention can be implemented in a modified form without departing from the essential characteristics of the present invention. Therefore, the disclosed embodiments should be considered from an illustrative point of view rather than a restrictive point of view. The scope of the present invention is indicated in the claims rather than the foregoing description, and all differences within the equivalent range will be construed as being included in the present invention.

## Claims

1. A pharmaceutical composition for preventing or treating a muscular disease, comprising an oxicam-based compound and a pharmaceutically acceptable salt thereof.

2. The pharmaceutical composition of claim 1, wherein the oxicam-based compound is selected from the group consisting of lornoxicam, piroxicam, tenoxicam, anpyroxicam, droxicam, meloxicam, chlornotexicam, tenoxicam, and piroxicam.

3. The pharmaceutical composition of claim 1, wherein the muscular disease is selected from the group consisting of atony, muscular atrophy, muscle degeneration, myotonia, amyotrophic sclerosis, myasthenia gravis, cachexia, and sarcopenia.

4. The pharmaceutical composition of claim 3, wherein the muscular disease is caused by aging, muscle dysfunction, muscle wasting, muscle degeneration, or muscle damage

5. A health functional food composition for preventing or alleviating a muscular disease, comprising an oxicam-based compound or a sitologically acceptable salt thereof.

6. A composition for strengthening muscle strength, comprising an oxicam-based compound.

7. A composition for improving muscle function, comprising an oxicam-based compound.

8. A composition for improving motor performance, comprising an oxicam-based compound.

9. The composition of any one of claims 6 to 8, wherein the composition is one or more selected from food, functional food, health functional food, pharmaceuticals, animal feed, or feed additives.

10. The pharmaceutical composition of any one of claims 6 to 8, wherein the oxicam-based compound is selected from the group consisting of lornoxicam, piroxicam, tenoxicam, anpyroxicam, droxicam, meloxicam, chlornotexicam, tenoxicam, and piroxicam.

11. A composition for promoting differentiation of muscular stem cells, comprising an oxicam-based compound.

12. A composition for muscle regeneration, comprising an oxicam-based compound.

13. A composition for increasing muscle mass, comprising an oxicam-based compound.

14. The composition of any one of claims 11 to 13, wherein the composition has an effect of muscular stem cell differentiation, muscle regeneration, or a muscle mass increase in damaged or aged muscle.

15. The composition of any one of claims 11 to 13, wherein the composition has an effect of muscular stem cell differentiation, muscle regeneration, or a muscle mass increase in normal muscle upon long-term administration.

16. The pharmaceutical composition of any one of claims 11 to 13, wherein the oxicam-based compound is selected from the group consisting of lornoxicam, piroxicam, tenoxicam, anpyroxicam, droxicam, meloxicam, chlornotexicam, tenoxicam, and piroxicam.

17. A method of preventing, alleviating, or treating a muscular disease by using an oxicam-based compound and a pharmaceutically acceptable salt thereof.

18. Use of an oxicam-based compound and a pharmaceutically acceptable salt thereof for preventing, alleviating, or treating a muscular disease
